# EUROPÄISCHE PATENTSCHRIFT

(11) **EP 2 644 143 B1**
(45) Veröffentlichungstag und Bekanntmachung des Hinweises auf die Patenterteilung: **08.03.2017**
(21) Anmeldenummer: 13159841.9
(22) Anmeldetag: 19.03.2013
(51) Int. Cl.: A61B 17/80, A61B 17/00, A61F 2/30

(54) **Antiinfektiver Abstandhalter für Osteosyntheseplatten**
Antiinfective spacer for osteosynthetic plates
Entretoise anti-infectieuse pour plaques d'ostéosynthèse

(30) Priorität: 30.03.2012 DE 102012006454
(43) Veröffentlichungstag der Anmeldung: 02.10.2013
(73) Patentinhaber: Heraeus Medical GmbH, 61273 Wehrheim (DE)
(72) Erfinder: Vogt, Sebastian, 99092 Erfurt (DE); Kühn, Klaus-Dieter, 35041 Marburg-Elnhausen (DE)
(74) Vertreter: Schultheiss & Sterzel Patentanwälte PartG mbB

(56) Entgegenhaltungen:
- EP-A1- 0 087 662
- EP-A2- 1 985 317
- WO-A1-88/03417
- WO-A1-2011/006228
- DE-A1- 19 511 268
- DE-U1-202005 020 490
- US-A1- 2006 293 670
- US-A1- 2007 173 840
- US-A1- 2008 097 432
- US-A1- 2009 082 816

## Beschreibung

Die Erfindung betrifft einen antiinfektiven Abstandhalter für Osteosyntheseplatten umfassend eine Platte mit mindestens einer Ausnehmung zur Aufnahme einer Schraube, sowie ein Set umfassend zumindest eine Osteosyntheseplatte und zumindest einen solchen Abstandhalter.

Unter dem Begriff Osteosynthese wird die operative Versorgung von Knochenbrüchen mit Implantaten verstanden, wobei zumeist Implantate aus biokompatiblen Metalllegierungen verwendet werden. Das Ziel der Osteosynthese ist die mechanische Fixierung von reponierten Knochenfragmenten während der Heilungsphase. Durch diese Fixierung wird die Wiederherstellung von Achsenstellungen und der Gelenkstellungen von frakturierten Knochen möglich.

Zur Osteosynthese werden neben Nägeln, Schrauben, Kirschnerdrähten besonders häufig Osteosyntheseplatten verwendet. Osteosyntheseplatten sind gelochte Platten aus biokompatiblen Metalllegierungen. Die Öffnungen in den Osteosyntheseplatten sind kreisrund oder oval. Die Osteosyntheseplatten werden mit den Enden der frakturierten Knochen nach deren Reposition verschraubt. Die Öffnungen der Osteosyntheseplatten sind so gestaltet, dass in diesen die Schraubenköpfe zumindest teilweise versenkt werden können. Dabei liegen die Osteosyntheseplatten unmittelbar zumindest teilweise auf dem Knochen auf.

Die Osteosyntheseplatten werden an beiden Enden der Knochenfragmente üblicherweise mit bikortikalen Schrauben festgeschraubt. Problematisch ist jedoch bei einer flächigen Auflage der Osteosyntheseplatten, dass auf Grund der Anpressung Nekrosen des Periosts (Knochenhaut) entstehen können. Deshalb wurden so genannte "Limited-contact-Plates" (LCP) entwickelt. Bei diesen Osteosyntheseplatten gibt es nur punktförmige oder streifenförmige Kontakte der Osteosyntheseplatten mit dem Periost. Das bedeutet, dass die Auflagefläche der Osteosyntheseplatten vermindert wird. Nachfolgend wurden so genannte "dynamic compression plates" (DCP), beziehungsweise "limited contact dynamic compression plates" (LC-DCP) entwickelt. Diese Platten enthalten ovale Öffnungen. Bei Verwendung von Schrauben mit ovalen Schraubenköpfen ist es möglich, beim Anziehen der Schrauben eine Kompression der Knochenfragmente gegeneinander zu erreichen. Gegenwärtig gibt es auch so genannte "winkelstabile" Osteosyntheseplatten. Bei diesen Platten aus speziellen Metalllegierungen ist unmittelbar unterhalb der Schraubenköpfe ein Gewinde angeordnet, so dass beim Anziehen der Schrauben an die Osteosyntheseplatte eine Kaltverschweißung der Schrauben mit der Platte erfolgt. Dadurch bildet die Osteosyntheseplatte mit den Schrauben eine Einheit und es wird ein mechanisch sehr stabiler Verbund ausgebildet.

Depots für die lokale Freisetzung von antimikrobiellen Wirkstoffen sind seit Jahrzehnten bekannt. So wird in der EP 0 170 979 A2 ein Depot auf Basis von Gelatine beschrieben. In der EP 0 087 662 A1 wird ein Wirkstoff-Depot offengelegt, das als Träger Tricalciumphosphat nutzt. Polymethylmethacrylat als Trägermaterial für antimikrobielle Wirkstoffe ist durch die Schriften DE 28 15 934 A1, US 4,191,743 und US 4,191,740 bekannt. Polymethylmethacrylat ist als Wirkstoffträger unter der Bezeichnung "Heraeus PMMA-Kette mit Gentamicin" von Heraeus Medical und als Septopal^{®} von der Firma Biomet eingeführt.

Aus der US 2008/097 432 A1 ist eine Osteosyntheseplatte bekannt, die mit einem Baumwollstoff laminiert ist. Die US 2009/082 816 A1 offenbart einen formbaren Abstandhalter zur Beabstandung einer Knochenplatte von einem Knochen, in dem ein antimikrobielles Polypeptid enthalten ist. Die WO 2011/006228 A1 offenbart einen verformbaren Abstandhalter für Osteosyntheseplatten, der sich an die Form des Knochens anpassen soll.

Aus der EP 2 005 978 B1 ist ein Abstandhalter für eine Knochenplatte bekannt, der eine Beschichtung mit einem Therapeutikum, einem Polymerträger und einem Puffermedium umfasst. Die Beschichtung soll die Knochenheilung und Knochenbildung anregen.

Nachteilig ist hieran, dass dieser Abstandhalter keine antimikrobielle Wirkung hat. Zudem ist die zeitliche Wirkung der Beschichtung begrenzt und nur bedingt steuerbar. Wünschenswert wäre zudem ein einfacher Herstellungsprozess, bei dem keine zusätzlichen Herstellungsschritte bei der Fertigung des Abstandhalters notwendig sind. Es besteht immer ein Bedürfnis nach einer kostengünstigen Fertigung.

Aufgabe der Erfindung ist es, diese und weitere nicht genannte Nachteile zu überwinden. Insbesondere soll eine zuverlässige und möglichst lang andauernde medizinische Wirkung der eingesetzten Abstandhalter erreicht werden. Gleichzeitig soll die Herstellung der Abstandhalter einfach und dabei kostengünstig sein. Aufgabe der Erfindung ist es ferner, einen antiinfektiven Abstandhalter für Osteosyntheseplatten zu entwickeln, der einen sicheren Abstand zwischen Osteosyntheseplatten und dem Knochen gewährleistet, um Drucknekrosen zu vermeiden.

Diese Aufgaben werden gelöst durch einen antiinfektiven Abstandhalter für Osteosyntheseplatten umfassend eine Platte mit mindestens einer Öffnung zur Aufnahme einer Schraube, bei dem die Platte aus mindestens einem biokompatiblen Material aufgebaut ist, in dem mindestens eine antimikrobielle Substanz verteilt, dispergiert oder gelöst ist, wobei der Abstandhalter auf mindestens einer Seite punktförmige oder streifenförmige Erhebungen besitzt und der Abstandhalter aus einem Kunststoff aufgebaut ist.

Die Verteilung oder Dispersion ist dabei im gesamten Material verteilt, vorzugsweise gleichmäßig im gesamten Material verteilt. Die Verteilung oder Dispersion kann dabei in Poren in dem Grundmaterial der Platte enthalten sein. Vorzugsweise sind die Poren im Wesentlichen komplett mit einer Substanz gefüllt, die die antimikrobiellen Substanzen enthält.

Dabei kann vorgesehen sein, dass zumindest eine solche Menge und/oder Konzentration an antimikrobieller Substanz in dem biokompatiblen Material enthalten ist, dass zumindest an der Oberfläche der Platte eine antimikrobielle Wirkung erzielbar ist, vorzugsweise in einem Bereich um die Oberfläche der Platte eine antimikrobielle Wirkung erzielbar ist.

Durch diese Maßnahme wird sichergestellt, dass die antiinfektiven Abstandhalter einen positiven keimhemmenden Effekt haben, wenn diese beim Patienten eingesetzt werden.

Durch die Erhebungen wird die Auflagefläche des Abstandhalters reduziert und damit die Gefahr von Knochennekrosen weiter verringert.

Es kann erfindungsgemäß auch vorgesehen sein, dass die Ausnehmungen kreisrund und/oder oval sind.

Durch diese Formen wird eine besonders einfache Handhabbarkeit auch während der Operation erreicht. Die ovale Form führt zu einer stärkeren Verbindung des Abstandhalters mit einer darin angezogenen Schraube.

Ferner kann vorgesehen sein, dass der Abstandhalter aus einem Kunststoff der Gruppe der Polymethylmethacrylate, Polymethylmethacrylat-co-methylacrylate oder Polymethylmethacrylat-co-styrene aufgebaut ist und/ oder zumindest ein Röntgenopaker in dem Kunststoff enthalten ist, vorzugsweise dispergiert ist, wobei der Röntgenopaker besonders bevorzugt aus der Gruppe des Bariumsulfats, des Zirkoniumdioxids und des Tantals ausgewählt ist.

Die Polymere aus der Gruppe der Polymethylmethacrylate, Polymethylmethacrylat-co-methylacrylate und Polymethylmethacrylat-co-styrene sind biokompatibel, wie auch die Verwendung in PMMA-Knochenzementen zeigt. Diese Kunststoffe können leicht durch übliche Formgebungsverfahren, wie Spritzgießen oder Pressen zu Platten geformt werden. Weiterhin ist es auch möglich, aus Plattenmaterial dieser Kunststoffe durch einfaches Stanzen unterschiedlich geformte Abstandhalter herzustellen. Besonders vorteilhaft ist es, wenn dem Kunststoff ein röntgenopakes Material beigemischt ist, um im Röntgenbild durch einen ausreichenden Kontrast gut erkennbar zu sein. Besonders geeignet sind dazu Bariumsulfat, Zirkoniumdioxid und Tantal. Es ist auch möglich, organische Jod-Verbindungen als röntgenopaker beizumischen. Durch den inkorporierten Röntgenopaker kann die Position der Abstandhalter problemlos röntgenologisch verfolgt werden.

Gemäß einer weiteren Ausgestaltung der Erfindung kann vorgesehen sein, dass der Abstandhalter aus einem Kunststoff aufgebaut ist und der Kunststoff ein die Wirkstofffreisetzung förderndes Additiv enthält, bevorzugt Calciumsulfat-Dihydrat, Calciumsulfat, Calciumcarbonat, Polyethylenglykol, Polyethylenglykol, Mannitol, Sobitol, Erythrol, Dianhydroglucitol, Anhydroglucitol, Glycin und/oder Alanin.

Durch das Additiv kann die Menge an antimikrobiellem Wirkstoff, der in dem Kunststoff gelöst oder dispergiert ist, reduziert werden oder die Freisetzung des antimikrobiellen Wirkstoffs kann verstärkt werden.

Gemäß einer weiteren, besonders bevorzugten Ausführungsform kann vorgesehen sein, dass der Abstandhalter ein poröses Metall umfasst, wobei die Poren in dem Metall interkonnektierend sind und bevorzugt einen Porendurchmesser von 1-300 µm aufweisen, besonders bevorzugt einen Porendurchmesser im Bereich von 1-100 µm aufweisen.

Das bedeutet, dass in den Hohlräumen der Poren antimikrobielle Substanzen eingelagert sein können beziehungsweise werden, die bei Kontakt mit Körperflüssigkeiten, wie Wundsekret oder Blut aus diesen heraus gelöst werden können beziehungsweise heraus lösbar sind. Die aus porösem Metall gefertigten Abstandhalter haben den Vorteil, dass sie eine sehr hohe Druckstabilität aufweisen.

Dabei kann vorgesehen sein, dass das poröse Metall Titan, eine Titanlegierung, Tantal, eine Tantallegierung oder Chrom-Kobalt-Stahl ist.

Diese Metalle eignen sich aufgrund ihrer guten Biokompatibilität und gleichzeitiger Elastizität besonders gut als Materialien für erfindungsgemäße Abstandhalter.

Ganz besonders bevorzugte und vorteilhafte Ausführungen der Erfindung zeichnen sich dadurch aus, dass vorgesehen ist, dass die antimikrobielle Substanz Antibiotika, Octenidindihydrochlorid, Polyhexanid, Chlorhexidin, Triclosan, quartäre Ammoniumsalze, Silbersalze, Silberoxid, Kupfer-Salze oder Kupferoxid oder eine Mischung dieser Substanzen ist.

Bei den Antibiotika sind insbesondere Aminoglykosid-Antibiotika wie Gentamicin, Tobramycin und Amikacin bevorzugt.

Diese Antibiotika können sowohl in Form leicht löslicher Salze, wie zum Beispiel der Sulfat-Form eingesetzt werden als auch in Form von in Wasser gering löslichen Salzen, wie zum Beispiel Fettsäuresalzen, Fettalkoholsulfaten oder Flavonphosphaten. Die Verwendung von gering löslichen Salzen der Antibiotika ermöglicht eine besonders bevorzugte retardierte Freisetzung dieser Antibiotika. Weiterhin verfügen dieses Antibiotika-Salze auch über ein gutes Haftvermögen auf metallischen und keramischen Oberflächen.

Aus der Gruppe der Antiseptika sind besonders Octenidindihydrochlorid, Polyhexanid, Chlorhexidin, Triclosan, quartäre Ammoniumsalze, Silbersalze, Silberoxid, Kupfer-Salze und Kupferoxid geeignet.

Gemäß einer weiteren Ausführungsform der Erfindung kann vorgesehen sein, dass der Gehalt der antimikrobiellen Substanz im Abstandhalter im Bereich von 0,1 bis 80 % ist, wobei der Bereich von 0,1 bis 40 % bevorzugt ist und der Bereich von 0,1 bis 20 % besonders bevorzugt ist.

Eine Ausführungsform eines erfindungsgemäßen Abstandhalters stellt eine Unterlegscheibe mit einem oder mehreren Ausnehmungen dar. Diese Unterlegscheibe kann kreisrund oder oval oder auch länglich in Form einer Lochplatte mit mehreren Ausnehmungen geformt sein.

Die Aufgaben der Erfindung werden auch gelöst durch ein Set umfassend zumindest eine Osteosyntheseplatte und zumindest einen solchen Abstandhalter, vorzugsweise umfassend wenigstens eine Schraube für jede Ausnehmung jedes Abstandhalters.

Das Set hat den Vorteil, dass gleich alle für eine Operation zur Behandlung der Fraktur des Knochens notwendigen Bauteile passend zueinander bereitgestellt werden.

Der Erfindung liegt die überraschende Erkenntnis zugrunde, dass es durch die Verwendung eines Materials als Abstandhandhalter, in dem eine antimikrobielle Substanz enthalten ist, die aus dem Material lösbar ist, gelingt, den Abstandhalter parallel zu seinen sonstigen Funktionen auch zur Vermeidung von Infektionen im Bereich der Abstandhalter, also am Knochen einzusetzen. Durch die andauernde Abgabe von antimikrobiellen Wirkstoffen wird auch die Vermeidung von Drucknekrosen unterstützt, beziehungsweise beim Auftreten von Drucknekrosen die Folgen dieser Komplikation abgemildert.

Der erfindungsgemäße Abstandhalter kann bei geeigneter Ausgestaltung über einen Zeitraum von mindestens mehreren Tagen wenigstens eine antimikrobiell wirksame Substanz abgeben, so dass mindestens die Oberfläche des Abstandhalters vor einer bakteriellen Besiedlung geschützt wird. Der Übergang vom Periost zur Kortikalis ist bei Plattenosteosynthesen besonders gegenüber Infektionen gefährdet, weil entlang der Schrauben mikrobielle Keime durch das Periost in die Kortikalis und dann in die Medula wandern können. Dadurch kann es zur Ausbildung einer Osteitis/Osteomyelitis kommen, bei der die Gefahr besteht, dass diese in den chronischen Zustand übergehen kann. Durch die erfindungsgemäßen Abstandhalter gelingt es nun insbesondere die Bereiche des Durchgangs der Schrauben vom Periost zur Kortikalis antimikrobiell zu schützen. Weiterhin ist der Abstandhalter biokompatibel und kostengünstig auf einfache Weise zu fertigen.

Der erfindungsgemäße Abstandhalter kann nicht nur bei Osteosyntheseplatten Verwendung finden. Es ist auch möglich diesen Abstandhalter bei sonstigen Osteosynthesmaterialien, bei den Schrauben eingesetzt werden, wie zum Beispiel bei Fixateuren extern einzusetzen.

Im Folgenden werden Ausführungsbeispiele der Erfindung anhand von vier schematisch dargestellten Figuren erläutert, ohne jedoch dabei die Erfindung zu beschränken. Dabei zeigt:
- Figur 1:: eine schematische perspektivische Ansicht eines erfindungsgemäßen Abstandhalters mit drei Ausnehmungen;
- Figur 2:: eine schematische perspektivische Ansicht eines alternativen erfindungsgemäßen Abstandhalters mit einer Ausnehmung;
- Figur 3:: eine schematische perspektivische Ansicht eines weiteren erfindungsgemäßen Abstandhalters mit einer Ausnehmung; und
- Figur 4:: eine Sicht auf Böden von Petrischalen enthaltend Bakterienrasen und erfindungsgemäße Abstandhalter zur Darstellung der antimikrobiellen Wirkung.

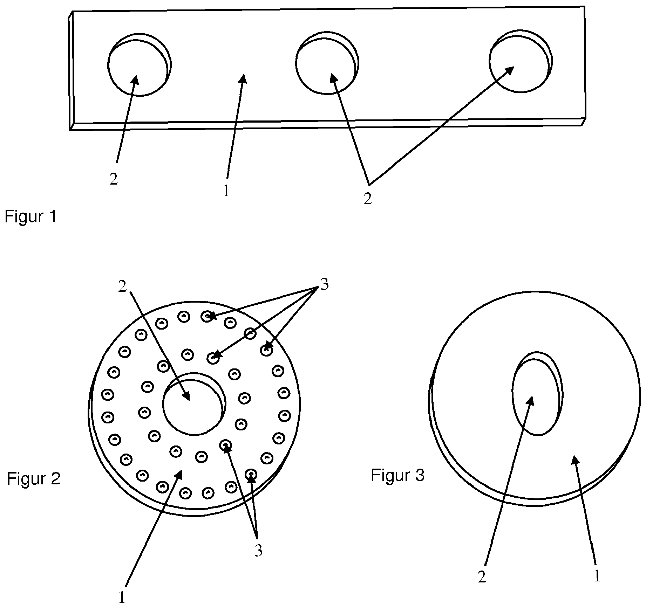

Figur 1 zeigt eine schematische perspektivische Ansicht eines erfindungsgemäßen Abstandhalters 1 mit drei Ausnehmungen 2. Der Abstandhalter 1 hat eine Dicke von 2,5 mm, ist 50 mm breit und 10 mm hoch. Die Ausnehmungen 2 sind äquidistant und sind kreisrund. Der Durchmesser der Ausnehmungen 2 beträgt 6 mm.

Der Abstandhalter 1 ist aus einer porösen Titanlegierung gefertigt, wobei die interkonnektierenden Poren einen durchschnittlichen Querschnitt von ungefähr 40 µm aufweisen. Die Poren sind im Wesentlichen vollständig mit einer Substanz gefüllt, die ein Antibiotikum oder ein Gemisch mehrerer Antibiotika umfasst.

Der Abstandhalter 1 wird bei einer Operation zwischen einer Osteosyntheseplatte und dem Knochen angeordnet, wobei die Schrauben, mit denen die Osteosyntheseplatte am Knochen befestigt wird, sich durch die Ausnehmungen 2 des Abstandhalters 1 erstrecken. Der Abstandhalter 1 beabstandet die Osteosyntheseplatte vom Knochen. Auf der Unterseite des Abstandhalters 1 ist eine leistenförmige Profilierung (nicht gezeigt) als streifenförmige Erhebungen vorgesehen, mit der der Abstandhalter 1 auf dem Knochen aufliegt.

Durch die Größe der Poren und durch die Zusammensetzung der Substanz selbst wird das oder die Antibiotika aus dem eingesetzten Abstandhalter 1 über die Zeit langsam aber stetig freigesetzt. Das Antibiotikum oder die Antibiotika werden durch die Wundsekrete und das Blut des Patienten aus dem Abstandhalter herausgelöst.

Die Figuren 2 und 3 zeigen alternative, erfindungsgemäße Abstandhalter 1 in einer schematischen, perspektivischen Ansicht. Die Abstandhalter 1 sind kreisrund nach Art einer Unterlegscheibe geformt. Der Abstandhalter 1 nach Figur 2 hat eine einzige zentrale, kreisrunde Ausnehmung 2. An der Oberfläche des Abstandhalters 1 ist eine Vielzahl von punktförmigen Erhebungen 3 vorgesehen, mit denen der Abstandhalter 1 im eingesetzten Zustand auf dem Knochen aufliegt. Durch diese Maßnahme soll eine möglichst geringe Kontaktfläche zur Vermeidung von Knochennekrosen erreicht werden. Der Abstandhalter 1 nach Figur 2 besteht aus einer porösen Aluminiumoxidkeramik, wobei in die durchschnittlich 50 ₁₁ m durchmessenden und untereinander verbundenen (interkonnektierenden) Poren eine antimikrobielle Substanz umfassend Chlorhexidin eingebracht ist.

Der Abstandhalter 1 nach Figur 3 umfasst eine ovale Ausnehmung 2. Die ovale Form der Ausnehmung 2 dient dazu, eine festere Verbindung des Abstandhalters 1 mit einer Schraube zu erreichen, mit der der Abstandhalter 1 und eine zugehörige Osteosyntheseplatte am Knochen eines Patienten befestigt wird. Der Abstandhalter 1 wird aus einem Kunststoff gefertigt, in dem eine antimikrobielle Substanz enthalten ist. Zusätzlich ist in dem Kunststoff ein die Wirkstofffreisetzung förderndes Additiv enthalten, das Calciumsulfat-Dihydrat umfasst.

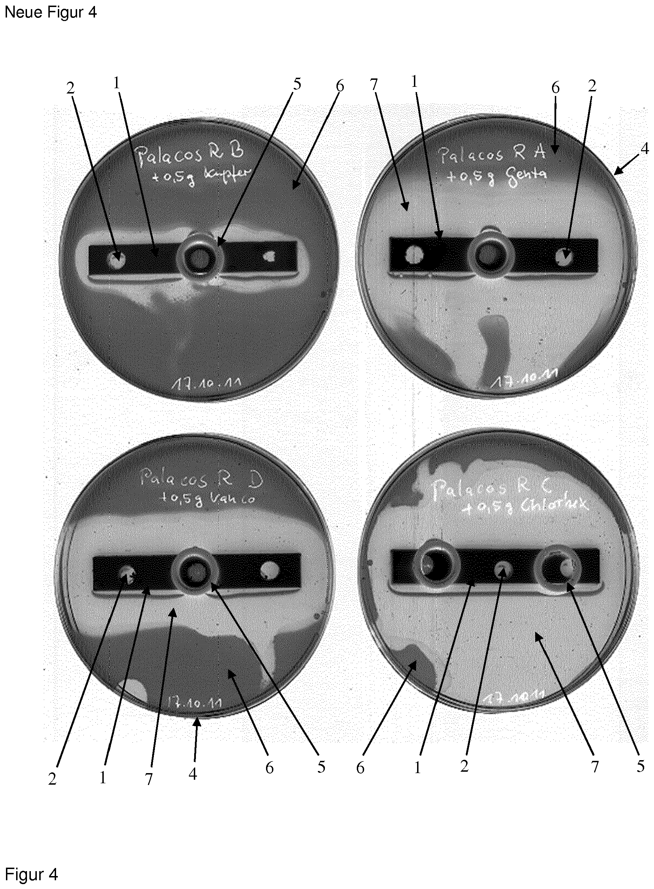

Figur 4 zeigt eine Aufsicht auf den Boden vier verschiedener Petrischalen 4, in denen vier verschiedene Abstandhalter 1 mit jeweils drei Ausnehmungen 2 auf Kunststoffrin gen 5 angeordnet sind. Die Kunststoffringe 5 beabstanden die Abstandhalter 1 vom Boden der Petrischale 4. Die Böden der Petrischalen 4 sind mit einem Bakterienrasen 6 (dunkler Randbereich in den Petrischalen 4) versehen, der zur Ausbildung eines Bakterienwachstums geeignet ist. Aufgrund der Wirkstofffreisetzung des antimikrobiellen Stoffs aus den erfindungsgemäßen Abstandhaltern 1 entstehen Hemmhöfe 7, in denen das Bakterienwachstum durch den antimikrobiellen Stoff unterdrückt oder verhindert wird.

Die Herstellung der vier erfindungsgemäßen Abstandhalter erfolgte wie folgt: Es wird zu 20 g Palacos R (Charge 7034) jeweils 0,5 g Wirkstoff zugegeben und dann in einer Kunststoffflasche zusammen mit drei Porzellan-Mahlkugeln mit Hilfe eine Turbula-Mischers 30 Minuten gemahlen. Anschließend werden diese Mischungen jeweils mit 10 ml Palacos-Monomerflüssigkeit vermischt und streifenförmige Abstandhalter 1 mit den Abmessungen 65 mm x 10 mm x 3,2 mm hergestellt, die jeweils drei Ausnehmungen 2 mit einem Durchmesser von 5 mm enthalten.

Die folgende Tabelle zeigt den Wirkstoffzusatz zu den vier verschiedenen Abstandhaltern 1:

| Beispiel | Wirkstoffzusatz |
|---|---|
| 1 (links oben) | 0,5 g Kupfer(II)sulfat (Sigma-Aldrich) |
| 2 (rechts oben) | 0,5 g Gentamicinsulfat (Fujian Fukang) |
| 3 (links unten) | 0,5 g Vancomycinhydrochlorid (Abbott) |
| 4 (rechts unten) | 0,5 g Chlorhexidindiacetat (Sigma-Aldrich) |

Zur Bestimmung der biologischen Wirksamkeit der Abstandhalter 1, werden in den Petrischalen 4 auf sterile Silikongummiringe 5 gelegt und mit einem CASO-Agar so umgossen, dass die Oberseite der Abstandhalter 1 aus dem Agar heraus ragte. Der CASO-Agar enthielt 106 CFU/ml einer Sporensuspension von bacillus subtilis ATCC 6633. Die Abstandhalter 1 auf dem CASO-Agar werden 20 Stunden bei 35 °C inkubiert. Zur Dokumentation wurden die Petrischalen 4 eingescannt und invertiert (siehe Figur 4), Hemmhöfe 7 erscheinen dann hell, der Bakterienrasen 6 dunkel.

Die Hemmhöfe 7 wurden gescannt und anschließend invertiert. Die Hemmhöfe 7 sind als helle Flächen erkennbar.

Bei den Beispielen 1 bis 4 wurde um jeden Abstandhalter 1 ein ausgeprägter Hemmhof 7 gefunden, wobei die Größe der Hemmhöfe 7 in der Reihenfolge Chlorhexidindiacetat, Gentamicin Vancomycin und Kupfer(II)sulfat abnahm.

Alle vier Beispiele für erfindungsgemäße Abstandhalter 1 sind also einsetzbar und als Abstandhalter 1 für Osteosytheseplatten auf Knochen einsetzbar.

### Bezugszeichenliste

- 1: Abstandhalter
- 2: Ausnehmung
- 3: Erhebung
- 4: Petrischale
- 5: Kunststoffring
- 6: Bakterienrasen
- 7: Hemmhof

## Patentansprüche

1. Antiinfektiver Abstandhalter (1) für Osteosyntheseplatten umfassend eine Platte mit mindestens einer Ausnehmung (2) zur Aufnahme einer Schraube, wobei die Platte aus mindestens einem biokompatiblen Material aufgebaut ist, in dem mindestens eine antimikrobielle Substanz verteilt, dispergiert oder gelöst ist, **dadurch gekennzeichnet, dass**
der Abstandhalter (1) auf mindestens einer Seite punktförmige oder streifenförmige Erhebungen (3) besitzt und der Abstandhalter (1) aus einem Kunststoff aufgebaut ist.

2. Abstandhalter (1) nach Anspruch 1, **dadurch gekennzeichnet, dass**
zumindest eine solche Menge und/oder Konzentration an antimikrobieller Substanz in dem biokompatiblen Material enthalten ist, dass zumindest an der Oberfläche der Platte eine antimikrobielle Wirkung erzielbar ist, vorzugsweise in einem Bereich um die Oberfläche der Platte eine antimikrobielle Wirkung erzielbar ist.

3. Abstandhalter (1) nach einem der vorangehenden Ansprüche, **dadurch gekennzeichnet, dass**
der Abstandhalter (1) aus einem Kunststoff der Gruppe der Polymethylmethacrylate, Polymethylmethacrylat-co-methylacrylate oder Polymethylmethacrylat-co-styrene aufgebaut ist.

4. Abstandhalter (1) nach einem der vorangehenden Ansprüche, **dadurch gekennzeichnet, dass**
zumindest ein Röntgenopaker in dem Kunststoff enthalten ist, vorzugsweise dispergiert ist.

5. Abstandhalter (1) nach Anspruch 4, **dadurch gekennzeichnet, dass**
der zumindest eine Röntgenopaker aus der Gruppe des Bariumsulfats, des Zirkoniumdioxids und des Tantals ausgewählt ist.

6. Abstandhalter (1) nach einem der vorangehenden Ansprüche, **dadurch gekennzeichnet, dass**
der Abstandhalter (1) aus einem Kunststoff aufgebaut ist und der Kunststoff ein die Wirkstofffreisetzung förderndes Additiv enthält, bevorzugt Calciumsulfat-Dihydrat, Calciumsulfat, Calciumcarbonat, Polyethylenglykol, Polyethylenglykol, Mannitol, Sobitol, Erythrol, Dianhydroglucitol, Anhydroglucitol, Glycin und/oder Alanin.

7. Abstandhalter (1) nach einem der Ansprüche 1 bis 4, **dadurch gekennzeichnet, dass**
der Abstandhalter (1) ein poröses Metall umfasst, wobei die Poren in dem Metall interkonnektierend sind und bevorzugt einen Porendurchmesser von 1-300 µm aufweisen, besonders bevorzugt einen Porendurchmesser im Bereich von 1-100 µm aufweisen.

8. Abstandhalter (1) nach Anspruch 7, **dadurch gekennzeichnet, dass**
das poröse Metall Titan, eine Titanlegierung, Tantal, eine Tantallegierung oder Chrom-Kobalt-Stahl ist.

9. Abstandhalter (1) nach einem der vorangehenden Ansprüche, **dadurch gekennzeichnet, dass**
die antimikrobielle Substanz Antibiotika, Octenidindihydrochlorid, Polyhexanid, Chlorhexidin, Triclosan, quartäre Ammoniumsalze, Silbersalze, Silberoxid, Kupfer-Salze oder Kupferoxid oder eine Mischung dieser Substanzen ist.

10. Abstandhalter (1) nach einem der vorangehenden Ansprüche, **dadurch gekennzeichnet, dass**
der Gehalt der antimikrobiellen Substanz im Abstandhalter (1) im Bereich von 0,1 bis 80 % ist, wobei der Bereich von 0,1 bis 40 % bevorzugt ist und der Bereich von 0,1 bis 20 % besonders bevorzugt ist.

11. Set umfassend zumindest eine Osteosyntheseplatte und zumindest einen Abstandhalter (1) nach einem der vorangehenden Ansprüche, vorzugsweise umfassend wenigstens eine Schraube für jede Ausnehmung (2) jedes Abstandhalters (1).

## Claims

1. Anti-infective spacer (1) for osteosynthesis plates comprising a plate having at least one recess (2) for accommodating a screw, whereby the plate is made up of at least one biocompatible material, in which at least one antimicrobial substance is distributed, dispersed or dissolved, **characterised in that**
the spacer (1) possesses point-shaped or strip-shaped elevations (3) on at least one side and the spacer (1) is made up of a plastic material.

2. Spacer (1) according to claim 1, **characterised in that**
at least a sufficient amount and/or concentration of antimicrobial substance is contained in the biocompatible material such that an antimicrobial effect can be attained at least at the surface of the plate, preferably an antimicrobial effect can be attained in a region extending beyond the surface of the plate.

3. Spacer (1) according to any one of the preceding claims, **characterised in that**
the spacer (1) is made up of a plastic material from the group of polymethylmethacrylates, polymethylmethacrylate-co-methylacrylates or polymethylmethacrylate-co-styrenes.

4. Spacer (1) according to any one of the preceding claims, **characterised in that**
the at least one radiopaquer is contained in the plastic material, preferably is dispersed in the plastic material.

5. Spacer (1) according to claim 4, **characterised in that**
the radiopaquer is selected from the group of barium sulfate, zirconium dioxide, and tantalum.

6. Spacer (1) according to any one of the preceding claims, **characterised in that**
the spacer (1) is made up of a plastic material and the plastic material contains an additive that enhances the release of the active substance, preferably calcium sulfate dihydrate, calcium sulfate, calcium carbonate, polyethylene glycol, mannitol, sorbitol, erythritol, dianhydroglucitol, anhydroglucitol, glycine and/or alanine.

7. Spacer (1) according to any one of the claims 1 to 4, **characterised in that**
the spacer (1) comprises a porous metal, whereby the pores in the metal are interconnecting and preferably have a pore diameter of 1-300 µm, particularly preferably have a pore diameter in the range of 1-100 µm.

8. Spacer (1) according to claim 7, **characterised in that**
the porous metal is titanium, a titanium alloy, tantalum, a tantalum alloy or chromium-cobalt steel.

9. Spacer (1) according to any one of the preceding claims, **characterised in that**
the antimicrobial substance is antibiotics, octenidine dihydrochloride, polyhexanide, chlorhexidine, triclosan, quarternary ammonium salts, silver salts, silver oxide, copper salts or copper oxide or a mixture of said substances.

10. Spacer (1) according to any one of the preceding claims, **characterised in that**
the content of the antimicrobial substance in the spacer (1) is in the range of 0.1 to 80 %, whereby the range from 0.1 to 40% is preferred and the range from 0.1 to 20 % is particularly preferred.

11. Set comprising at least one osteosynthesis plate and at least one spacer (1) according to any one of the preceding claims, preferably comprising at least one screw for each recess (2) of each spacer (1).

## Revendications

1. Entretoise anti-infectieuse (1) pour plaques d'ostéosynthèse comprenant une plaque avec au moins un évidement (2) pour la réception d'une vis, où la plaque est constituée d'au moins un matériau biocompatible dans lequel est distribuée, dispersée ou solubilisée, au moins une substance antimicrobienne, **caractérisée en ce que**
l'entretoise (1) possède des élévations (3) en forme de points ou de bandes sur au moins une face et l'entretoise (1) est constituée d'une matière synthétique.

2. Entretoise (1) selon la revendication 1, **caractérisée en ce**
**qu'**au moins une quantité et/ou concentration en substance antimicrobienne telle est contenue dans le matériau biocompatible qu'un effet antimicrobien peut être atteint au moins sur la surface de la plaque, de préférence, un effet antimicrobien peut être atteint dans une région autour de la surface de la plaque.

3. Entretoise (1) selon l'une des revendications précédentes, **caractérisée en ce que**
l'entretoise (1) est constituée d'une matière synthétique du groupe des polyméthylméthacrylates, des polyméthylméthacrylate-co-méthylacrylates ou des polyméthylméthacrylate-co-styrènes.

4. Entretoise (1) selon l'une des revendications précédentes, **caractérisée en ce qu'**au moins un opacifiant de rayons X est contenu dans la matière synthétique, de préférence dispersé.

5. Entretoise (1) selon la revendication 4, **caractérisée en ce que**
l'au moins un opacifiant de rayons X est choisi dans le groupe du sulfate de baryum, du dioxyde de zirconium et du tantale.

6. Entretoise (1) selon l'une des revendications précédentes, **caractérisée en ce que**
l'entretoise (1) est constituée d'une matière synthétique et la matière synthétique contient un additif favorisant la libération de matière active, de préférence du sulfate de calcium di hydraté, du sulfate de calcium, du carbonate de calcium, du polyéthylène glycol, du mannitol, du sorbitol, de l'érythrol, du dianhydro glucidol, de l'anhydro glucidol, de la glycine et/ou de l'alanine.

7. Entretoise (1) selon l'une des revendications 1 à 4, **caractérisée en ce que** l'entretoise (1) comprend un métal poreux, où les pores dans le métal sont interconnectées et présentent de préférence un diamètre de pores dans la plage de 1 à 300 µm, présentent de préférence particulièrement un diamètre de pores dans la plage de 1 à 100 µm.

8. Entretoise (1) selon la revendication 7, **caractérisée en ce que** le métal poreux est du titane, un alliage de titane, du tantale, un alliage de tantale ou un acier chrome-cobalt.

9. Entretoise (1) selon l'une des revendications précédentes, **caractérisée en ce que**
la substance antimicrobienne est un antibiotique, du dichlorure d'octénidine, du polyhexanide, de la chlorhexidine, du triclosan, des sels d'ammonium quaternaire, des sels d'argent, de l'oxyde d'argent, des sels de cuivre ou de l'oxyde de cuivre ou un mélange de ces substances.

10. Entretoise (1) selon l'une des revendications précédentes, **caractérisée en ce que**
la teneur en substance antimicrobienne dans l'entretoise (1) est dans la plage de 0,1 à 80 %, où la plage de 0,1 à 40 % est préférée et la plage de 0,1 à 20 % est particulièrement préférée.

11. Ensemble comprenant au moins une plaque d'ostéosynthèse et au moins une entretoise (1) selon l'une des revendications précédentes, comprenant de préférence au moins une vis pour chaque évidement (2) de chaque entretoise (1).
